Europäisches Patentamt

(19) European Patent Office

Office européen des brevets

(11) Publication number: **0 104 793**
**A1**

(12) **EUROPEAN PATENT APPLICATION**

(21) Application number: **83305025.5**

(22) Date of filing: **31.08.83**

(51) Int. Cl.³: **A 61 N 1/42**
**A 61 D 9/00**

(30) Priority: **31.08.82 US 413539**

(43) Date of publication of application:
**04.04.84 Bulletin 84/14**

(84) Designated Contracting States:
**AT BE CH DE FR GB IT LI LU NL SE**

(71) Applicant: **ELECTRO-BIOLOGY, INC**
**300 Fairfield Road**
**Fairfield New Jersey 07006(US)**

(72) Inventor: **Pilla, Arthur A.**
**133 Heights Road**
**Ridgewood New Jersey 07450(US)**

(72) Inventor: **Ryaby, John P.**
**42 Stewart Road**
**Essex Fells New Jersey 07021(US)**

(72) Inventor: **Talish, Roger J.**
**12 Knoll Road**
**Fairfield New Jersey 07006(US)**

(74) Representative: **Wright, Peter David John et al,**
**R.G.C. Jenkins & Co. 12-15, Fetter Lane**
**London EC4A 1PL(GB)**

(54) Apparatus for equine limb treatment.

㉗ The invention contemplates an assembly of two coils (22,30) adapted for removable attachment to an equine limb, and containing its own power source and pulse-signal generator, serving both coils in flux-aiding relation. The pulse-signal generator excites the coils with a succession of therapeutically beneficial low-voltage unidirectional asymmetrical pulses.

EP 0 104 793 A1

Croydon Printing Company Ltd.

- 1 -

# APPARATUS FOR EQUINE LIMB TREATMENT

## Background of the Invention

The invention relates to an electromagnetic body-treatment device for surgically non-invasive modification of the growth, repair and maintenance behaviour of living tissues and/or cells within an equine or the like limb, by a specific and selective change in electrical environment.

Applicable background is discussed at length in Ryaby, et al., U.S. Patent No. 4,105,017 and in various patents having a continuation-in-part relation thereto. Reference is therefore made to said patents for background discussion. It suffices to note that, although said patents speak generally in the context of controlled modification of cellular and/or tissue growth, the primary emphasis on osteogenic applications to human beings, was initially limited to such "Hopeless" cases as pseudarthroses and other non-unions.

Devices used on human beings for osteogenic growth and repair have followed the teachings of Ryaby, et al., Patent No. 4,266,532, particularly the embodiment of Fig. 7 of said patent, wherein two electrical coils are positioned on opposite sides of a fracture and are excited in flux-aiding relation to develop a substantially uniform zone of magnetic-flux concentration in which the afflicted body region is immersed. The power source of pulse-excitation-signals to the coils is external and bulky, and the signals have followed magnitude and other parameter limitations, based on limitations having FDA approval.

In an attempt to apply human-being experience to horses, an immediate problem is encountered in that a horse will not willingly permit attempted treatment with a device which can be kicked or destroyed or which requires connection to an external power source.

- 3 -

BRIEF STATEMENT OF THE INVENTION:

It is an object of the invention to provide improved applicator means, removably adapted for the pulsed electromagnetically stimulated treatment of a limb-related injury of the character indicated.

It is a specific object to provide a completely self-contained device of the character indicated which can be applied to an equine limb, which is of negligible weight and inconvenience to the animal and which may therefore be left in place on the afflicted limb, with selective availability of the control of treatment in relation to non-treatment intervals within a given day.

The invention achieves the foregoing objects in a device employing two coils and attachment structure generally as described in said Patent No. 4,226,532, but containing its own source of power and pulse-generating circuitry, and involving pulse-signal energy levels that are well below those in current human application

- 4 -

and below those set forth in said patent; in addition, certain other signal parameters depart from values and value ranges previously set forth. The device is light in weight and of little bulk and is easily tolerated by the animal, and its therapeutic benefits are achieved while the animal is ambulatory. Impressive results are achieved in aid of rapid recovery by horses, particularly race horses, from the kinds of bone and bone-related injury variously known as saucer fractures, damaged suspensories, splints and bucks; and non-bone afflictions such as bowed tendons have been successfully and rapidly treated. Additionally, the injured animal need no longer be subjected to the pain of blistering (chemical burning) or firing (red-hot pin insertion through the skin), nor need he be subjected to the long recovery period or curtailed career (in the case of a race horse), which have hitherto been accepted as necessary consequences of injuries of the character indicated.

DETAILED DESCRIPTION

The invention will be described for an illustrative embodiment in conjunction with the accompanying drawings, in which:

Fig. 1 is a simplified view in perspective showing a device of the invention applied to the lower region of an injured equine metacarpus;

Fig. 2 is an enlarged plan view of the device of Fig. 1, prior to assembly to a body limb and as viewed from the outside aspect, i.e., the aspect which, after application to a limb, will be from the outside, looking in;

Fig. 3 is a sectional view, taken at 3-3 in Fig. 2; and

Fig. 4 is a simplified diagram, on a reduced scale, showing one assembled portion of the device of Fig. 1, as viewed from the inside aspect (i.e., opposite to the aspect of Fig. 2), to illustrate features of selective adaptation to various sizes of

- 6 -

afflicted limb.

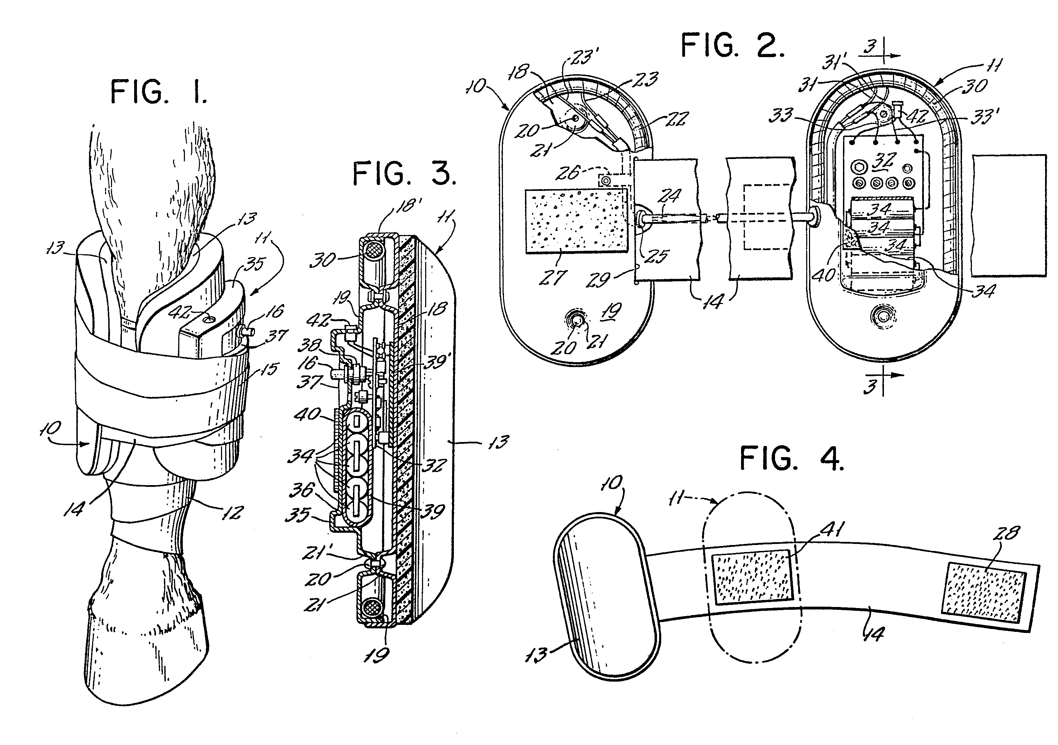

In Fig. 1, the device of the invention is shown in application to the lower region of a horse's metacarpus, which illustratively may have suffered one of the above-indicated bone injuries. Two electrical coil units 10-11 have been applied in diametrically opposed relation to a bandage wrap 12 around the afflicted region. Each coil unit has a lining 13 of soft cushioning material, as of urethane foam, and a circumferential elastic strap 14 compress- ionally loads the coil units against bandage 12. An outer bandage wrap 15 assures against loss of the setting of elastic strap 14. The respective coils within units 10-11 are driven by a pulse generator and its power supply, both contained within unit 11, and an externally accessible electrical jack 16 acts as a switch, automatically turning-on the pulse generator (when jack 16 is removed from its receptacle) and automatically switching-off the pulse generator (when jack 16 is inserted into its receptacle). Also, when jack 16 is removed, the jack receptacle will be understood to provide plug-in access for battery-recharging voltage to be served to the internal battery, from an external charging source (not shown).

In Figs. 2 and 3, units 10 and 11 are seen to be of like nature and construction, except for the fact that the power source and pulse-signal generating means are carried only at unit 11. Unit 10 comprises two fitted casing halves 18-19 of suitable plastic material, having telescoping fit of their respective peripheral flanges 18'-19', and secured to each other as by rivets 20 at abutting pedestal formations 21-21'. The telescoped flanges are contour-conforming to a multiturn electrical coil 22, shown tape-wrapped and nested within the flanges 18'-19'. Coil shape is generally elliptical, to a major-axis extent which may be approximately twice the minor-axis extent. Coil shape is also characterized by generally cylindrically arcuate curvature to a radius of approximately minor-axis extent, about a cylinder axis parallel to the major elliptical axis. The fitted casing halves 18-19 conform to and retain these coil-curvature characteristics.

For convenience, the casing half 18, which is characterized by a concave generally cylindrical outer surface, will be referred to as the inner half, and the casing half 19, having a convex outer surface, will be referred to as the outer half.

Electrical leads 23-23' to coil 22 are connected, within the casing 18-19, to conductors of a flexible cable 24. Cable 24 extends between units 10-11 and enters unit 10 via a port formation 25 near the center of one of the long edges of outer casing half 19, being clamped at 26 to avoid stress on the cable-to-coil connection. The end of strap 14 which is fixed to unit 10 passes through a slot opening 29 in casing half 19, and is welt-reinforced within the casing to assure against loss through the slot. Finally, a large central rectangular piece 27 of detachable coupling fabric, such as a loop fabric of Velcro material, is secured to the convex outer surface of casing half 19, for releasable engagement to a piece 28 of coacting hook fabric

0104793

- 9 -

secured to the free end of strap 14.

Those features of unit 11 which are the same as for unit 10 are shown with the same reference numbers. The two casing halves of unit 11 contain and peripherally retain the elliptical shape of the second coil 30 which may be identical with coil 22 and which is shown with its leads 31-31' connected (via cable 24) in parallel to those of coil 22. A printed-circuit board 32 is contained within the casing of unit 11 and carries pulse-generator circuitry having output connection via leads 33-33' to the line of electrically parallel connection of coils 22-30; input power to the pulse-generator circuitry is provided by small rechargeable batteries 34, shown as five AA-size cells, connected in series and also contained within the casing of unit 11; a protective wrap 39 of bandage or the like fabric material helps retain the assembled relation of battery cells and cushions them against displacement within the casing, and a protective pad 39' of suitably shock-absorbent material protects pulse-generator components from direct mechanical-shock contact with adjacent casing of unit 11. The external contour of the outer casing half 19 of unit 11 will be seen to include a more pronounced bulge 35, to accommodate self-contained battery and circuit components. The bulge has an outwardly projecting rim, surrounding a recessed panel portion 36 overstanding the batteries and a further recessed region 37 where a jack receptacle 38 is mounted for jack (16) reception. Finally, a rectangular piece 40 of loop fabric is secured to the outer convex surface of panel region 36, for selective removably attached engagement to a piece 41 of hook fabric fixed to the middle range of strap 14, and an indicator lamp such as an LED device 42, mounted to the upper side of the rim of bulge 35, provides externally viewable confirmation of whether or not the pulse generator is operating.

The Ryaby, et al. patents referred to above describe value ranges for various parameters of applied signals. Generally, the signals found effective for equine treatment involve energy and frequency levels at or below lower limits of ranges specified in said patents. Specifically, for equine applicators of the type herein described, it is found effective to provide asymmetrical pulse excitation of the described coils. In most of the clinical equine work to date, the pulses are of quasi-rectangular form, comprising a first pulse portion of relatively low-magnitude first polarity and of approximately 250-microsecond effective duration, followed by a second pulse portion of relatively greater magnitude second polarity and of approximately 4-microsecond effective duration. Such pulses are repeated in bursts of 30 milliseconds, but about 4kHz, and the bursts are repeated at a repetition rate of 1.5Hz. The amplitude of excitation signal is such as to develop a maximum body-indiced voltage of approximately 0.2 millivolt per centimeter of treated tissue and/or cells, as measured by a probe coil of the type described in said Ryaby et al. patents. Stated more generally, present experience indicates that, for the stated pulse signals, the burst-repetition rate should be in the range of 1 to

- 12 -

5Hz, that the pulses within each burst should be in the range of 2 to 5kHz, and that the burst duration should be approximately one fifth to one fiftieth the period of burst repetition. The involved maximum magnetic-field strength is approximately 2 Gauss during the first pulse portion. For the described parallel connection of coils 22-30, each coil may suitably comprise 80 turns of 19 AWG enameled copper wire, to an initial diameter of 18-cm, and after winding, each coil is bent to its above-described shape.

In use, the installed spacing of units 10-11 on strap 14 will depend upon girth of the afflicted limb region. The removable engagement at 40 (on unit 11) and at 41 (midway along strap 14) enables adjustment of the secured relative position of units 10-11 until their desired diametrically opposed relation has been established, whereupon the remaining free end of strap 14 may be tensed to the desired degree before anchoring the free-end hook material to the loop material on the outer panel 36 of unit 11.

0104793

- 13 -

Use of the described invention appears likely to revolutionize the repair therapy of bone fractures which frequently occur with race horses. Significant racing events, such as the Belmont and the Preakness have in 1982 been won by horses whose prior limb fractures had been aided in repair by equipment of the invention. The prior fractures of these winning horses were of a nature such that, with conventional therapy, it would be an open question whether the horse would ever again be able to race, and certainly the at-least one year previously considered necessary for bone repair in each case would have precluded participation in any post-fracture 1982 event, so that entry in either of these prestigious events would have been out of the question.

At this point in time, insufficient data have been accumulated to enable a full explanation of the physical process involved in body-cell response to the indicated pattern of pulsed electromagnetic fields. However, it is becoming clear that the device of the invention materially accelerates the healing process; a horse with a limb fracture of the character indicated can be back in training within the hitherto unheard-of short period of 30 to 60 days. The applicators are applied in medial-lateral relation to the limb (as in Fig. 1) to allow articulation. The pulsed electromagnetic therapy brings blood to the afflicted region, and the described coils appear to have an analgesic effect, in that pain disappears in 5 to 10 days. The treatment is illustratively applied for two hours per day until there is an X-ray appearance of healing; an X-ray look every three weeks is recommended.

Quite aside from use of the described device is post-fracture bone-healing applications, it is becoming apparent that regular use of the device on an apparently healthy horse produces desirable prophylactic results. We speculate that an apparently healthy horse may have a "hairline" or incipient fracture condition which is not X-ray discernable and is certainly not known to his trainer. Such a horse is thus accident-prone, but this fact is not known.

However, with regular prophylactic use, say for an hour each day while in pasture, particularly after an exhausting workout or a race, the horse is kept in best condition for racing without injury.

Although the above-described coils and pulse-signal parameters have developed an impressive record of successful treatment of race horses, further development indicates the achievement of superb clinical results using significantly reduced energy, longer battery life, and reduced weight. In this development, the coils 22-30 are series-connected, each of them being 16 turns of 22-AWG wire. The involved pulse signals comprise a first-polarity low-magnitude pulse of approximately 20-microsecond effective duration, and a second-polarity pulse of relatively greater magnitude and approximately 4-microseconds duration. Pulses within each burst are at 33kHz, and the burst-repetition rate is 2Hz, amplitudes being such as again to develop a maximum magnetic-field strength of approximately 0.5 Gauss during the first polarity pulse. When sufficiently tested, this low-energy pulse-signal pattern may become our preferred pattern of excitation, and we believe that therapeutic utility exists for even shorter second-polarity pulses and for pulse repetition with each burst at frequencies as high as 100kHz or more, with burst repetition within the indicated 1 to 5Hz range.

Recent experimental evidence indicates that therapeutically beneficial results are achievable with periodic repetition of a single-pulse signal at low-energy levels applicable to self-contained devices of the above-described nature and therefore suitable for equine and other animal bone-fracture repair. This evidence pertains to observations on enzyme activity in body cells, in response to electromagnetic stimulation with described coil configurations, the particular enzyme being adenosine triphosphatase (otherwise known as Na-K ATPase), and the observed result has been a significant increase in the rate of sodium-ion eflux and potassium-ion influx, within the region of treatment. These enhanced rates importantly relate to bone-fracture repair, and in particular to the favourable treatment of the edema (or inflammationary swelling) surrounding a bone injury; not only is the pain materially reduced in intensity and duration, but favourable initiation of a bone-repair environment is more rapidly established. The single-pulse signals involved are asymmetric and at a repetition rate in the range of 1 to 5Hz, the first portion of each pulse being of approximately 20-microsecond duration, and the second portion of each pulse being of approximately 2-microsecond duration, while energy amplitudes are such as to produce a maximum body-induced voltage of approximately 0.1 to 0.5 millivolt per centimeter of treated tissue and/or cells.

While the invention has been described in detail for a preferred embodiment, it will be understood that modifications may be made without departing from the scope of the invention.

CLAIMS:

1. An electromagnetic body-treatment device for surgically non-invasive modification of the growth, repair and maintenance behaviour of living tissues and/or cells within a limb of a living body by a specific and selective change in electrical environment, comprising two like multi-turn electrical coils of generally elliptical configuration having an elongate major axis and a relatively short minor axis, a coil casing of magnetically transparent electrically insulating material containing each coil and retaining the same in conformance with a generally cylindrically arcuate surface wherein the minor axis of the coil is conformed to the curvature of the cylindrical arc, removably securable flexible-strap means for connecting the coil casings to each other in parallel orientation of coil major axes, said strap means and connected casings being adapted to establish a circumferentially complete hoop wherein said casings are generally diametrically opposed with the concave sides of said casings facing each other, flexible electrical means connecting said coils in flux-aiding relation, and self-contained means within at least one of said casings and connected to said coils for electrically exciting said coils with a succession of low-voltage unidirectional asymmetrical pulses.

2. The treatment device of claim 1, in which said removably securable adjustable strap means is sized to adapt said coils to an equine limb.

3. The treatment device of claim 1, in which said pulses are in bursts at a burst-repetition rate in the range of 1 to 5Hz, wherein pulses within each burst are in the repetition-rate range of 2 to 5kHz, wherein burst duration is approximately one-fifth to one-fiftieth the period of burst repetition, and wherein the maximum body-induced voltage is approximately 0.2 millivolt per centimeter of treated tissue and/or cells.

4. The treatment device of claim 1, in which said pulses are in bursts at a repetition rate of about 1.5Hz, wherein pulses within each burst are at a repetition rate of 3 to 4kHz, wherein burst duration is approximately one-twentieth the period of burst repetition, and wherein the maximum body-induced voltage is approximately 0.2 millivolt per centimeter of treated tissue and/or cells.

5. The treatment device of claim 1, in which said self-contained means comprises a storage battery and a generator of electrical voltage pulses.

6. The treatment device of claim 5, in which said storage battery is rechargeable, and an externally accessible jack receptacle carried by one of said casings and conne:ted to said battery for removable connection thereof to a battery charger external to said device.

7. The treatment device of claim 5, in which one of said casings carries an externally accessible switch having on-off control of said excitation, and an externally viewable indicator of operational state.

8. The treatment device of claim 5, in which said battery and pulse generator are both contained within the same casing.

9. The treatment device of claim 1, in which for each coil the major axis span is approximately twice the minor axis span.

10. The treatment device of claim 9, in which the radius of arcuate cylindrical curvature is of approximately the magnitude of the minor-axis span.

11. The treatment device of claim 1, in which a liner of softly compressible material lines the concave side of each casing.

12. The treatment device of claim 1, in which said strap means includes elastic spans between said casings.

13. The treatment device of claim 1, in which said strap means comprises a single length of elastic strap secured at one end to one of said casings; first detachably securable means including a piece of hook-characterized fabric and a piece of loop-characterized fabric, one of said pieces being secured to the convex side of said one casing and the other of said pieces being secured to the other end of said strap; and second detachably securable means including a piece of hook-characterized fabric and a piece of loop-characterized fabric, one of the pieces of said last-defined means being secured to the convex side of the other casing, and the other piece of said last-defined means being secured to said strap substantially midway between the ends of said strap; whereby said strap may connect said casings in such adjustably spaced array as to enable application of said device to limbs of different girth, with assurance of mutually opposed elastically loaded retention of said coils.

14. The treatment device of claim 13, in which said battery and pulse generator are both contained within said other casing.

15. The treatment device of claim 2 or claim 3, in which said unidirectional asymmetric pulses comprise a relatively low-magnitude first pulse portion of first polarity and of approximately 250-microsecond effective duration, followed by a second pulse portion of relatively great-magnitude second polarity and of approximately 4-microsecond effective duration, and in which the involved maximum magnetic-field strength within the geometric volume defined by and between said coils during the first pulse portion in application to an animal limb is approximately 2 Gauss.

16. The treatment device of claim 1, in which said pulses are in bursts at a burst-repetition rate in the range of 1 to 5Hz, wherein pulses within each burst are in the repetition-rate range of 5 to 500kHz, wherein burst duration is approximately one-tenth the period of burst repetition, and wherein the maximum body-induced voltage is approximately 0.4 millivolt per centimeter of treated tissue and/or cells.

0104793

- 24 -

17.  The treatment device of claim 1, in which said pulses are in bursts at a repetition rate of about 2Hz, wherein pulses within each burst are at a repetition rate of 30 to 40kHz, wherein burst duration is approximately one-tenth the period of burst repetition, and wherein the maximum body-induced voltage is approximately 0.4 millivolt per centimeter of treated tissue and/or cells.

18.  The treatment device of claim 16 or claim 17, in which said unidirectional asymmetric pulses comprise a relatively low-magnitude first pulse portion of first polarity and of approximately 20-microsecond effective duration, followed by a second pulse portion of relatively great-magnitude second polarity and of approximately 4-microsecond effective duration, and in which the involved maximum magnetic-field strength within the geometrical volume defined by and between said coils during the first pulse portion in application to an animal limb is approximately 0.5 Gauss.

19. The treatment device of claim 1, in which said pulses are single asymmetric pulses having a repetition rate in the range of 1 to 5Hz, and wherein the maximum body-induced voltage is approximately 0.1 to 0.5 millivolt per centimeter of treated tissue and/or cells.

FIG. I.

FIG. 3.

FIG. 2.

FIG. 4.

1/1

0104793

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y,D | US-A-4 266 532 (RYABY) <br><br> * Figures 5a,b,7; column 8, lines 15-43 * | 1-4,15 -19 | A 61 N 1/42 <br> A 61 D 9/00 |
| Y | US-A-4 153 009 (BOYLE) <br><br> * Column 2, lines 30-31; column 17, line 64 - column 18, line 18 * | 1-4,15 -19 | |
| A | US-A-4 315 503 (RYABY) <br><br> * Column 17, line 64 - column 18, line 18 * | | |
| T | IEEE PROCEEDINGS, vol. 128, Pt. A, no. 5, July 1981, pages 329-335, Old Woking, Surrey, GB J. WATSON et al.: "Electricity and bone healing" * | | **TECHNICAL FIELDS SEARCHED (Int. Cl. ³)** <br><br> A 61 D <br> A 61 N |
| T | MEDICAL & BIOLOGICAL ENGINEERING & COMPUTING, vol. 20, no. 4, July 1982, pages 501-511, Stevenage, Herts, GB M.J. LUNT: "Magentic and electric fields produced during pulsed-magnetic-field therapy for non-union of the tibia" | | |
| | --- -/- | | |

The present search report has been drawn up for all claims

| Place of search THE HAGUE . | Date of completion of the search 06-12-1983 | Examiner GLAS J. |
|---|---|---|

## European Patent Office

### EUROPEAN SEARCH REPORT

| DOCUMENTS CONSIDERED TO BE RELEVANT | | Page 2 |

| Category | Citation of document with indication, where appropriate, of relevant passages | Relevant to claim | CLASSIFICATION OF THE APPLICATION (Int. Cl. ³) |
|---|---|---|---|
| T | PROC. OF THE IEEE, vol. 67, no. 9, September 1979, pages 1339-1352, IEEE, New York, US J. WATSON: "The electrical stimulation of bone healing" ----- | | |
| | | | TECHNICAL FIELDS SEARCHED (Int. Cl. ³) |

The present search report has been drawn up for all claims

| Place of search | Date of completion of the search | Examiner |
|---|---|---|
| THE HAGUE | 06-12-1983 | GLAS J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82